(19) Europäisches Patentamt
European Patent Office
Office européen des brevets

(11) EP 0 943 322 B1

(12) FASCICULE DE BREVET EUROPEEN

(45) Date de publication et mention
de la délivrance du brevet:
**24.09.2003 Bulletin 2003/39**

(51) Int Cl.⁷: **A61K 7/48**

(21) Numéro de dépôt: **99400044.6**

(22) Date de dépôt: **08.01.1999**

(54) **Utilisation d'une composition cosmétique pour le traitement et la prévention de la rosacée**

Verwendung einer kosmetischen Zusammensetuzung zur vorbeugung und behandlung vom Rosazea

Use of a cosmetic composition to prevent and treat the rosacea

(84) Etats contractants désignés:
**AT BE CH DE ES FR GB IT LI NL SE**

(30) Priorité: **03.02.1998 FR 9801220**

(43) Date de publication de la demande:
**22.09.1999 Bulletin 1999/38**

(73) Titulaire: **LA ROCHE POSAY Laboratoire
Pharmaceutique**
**86270 La Roche Posay (FR)**

(72) Inventeurs:
• **Richard, Alain**
**44600 Saint Nazaire (FR)**
• **Brissonnet, Jean-Pierre**
**86210 Vouneuil sur Vienne (FR)**

(74) Mandataire: **Renard, Emmanuelle**
**L'OREAL/DPI**
**6, Rue Bertrand Sincholle**
**92585 Clichy Cedex (FR)**

(56) Documents cités:
**EP-A- 0 761 204       EP-A- 0 775 492**

• **M. DE LA BRASSINNE: "Approche
Therapeutique de l'Acne" REVUE M DICALE DE
LI GE, vol. 39, no. 8, 1984, pages 305-307,
XP002082711**
• **G. GUILLET: "Traitement de l'Acne" BORDEAUX
M DICAL, vol. 16, no. 3, 1983, pages 131-132,
XP002082712**

## Description

L'invention a pour objet l'utilisation pour la prévention et/ou le traitement de la rosacée d'une composition cosmétique comprenant au moins de 1 à 5 % d'un acide gras en $C_{12}$-$C_{24}$, de 5 à 15 % d'un ester d'acide gras en $C_{12}$-$C_{24}$ et d'un fragment polyoxyde d'alkylène en $C_2$-$C_3$ comportant de 2 à 100 résidus polyoxyde d'alkylène, de 1 à 20 % d'un glycéride d'acide gras en $C_{12}$-$C_{22}$ éventuellement polyoxyalkyléné comportant de 0 à 20 résidus oxyde d'éthylène, de 1 à 20 % d'un ester d'un acide gras en $C_{12}$-$C_{24}$ et d'un alcool en $C_1$-$C_6$, de 0,1 à 10 % de glycérol, de 0,1 à 20 % d'un ester d'un acide gras en $C_{12}$-$C_{24}$ et d'un alccol en $C_1$-$C_6$, de 0,1 à 10 % de glycérol, de 0,1 à 3 % d'un alcool gras en $C_{12}$-$C_{24}$, cette composition comprenant de l'eau et ne comprenant pas de métronidazole.

La rosacée, appelée également de façon impropre acné rosacée, est une dermatose fréquente, touchant surtout le visage chez la femme entre 30 et 50 ans, dont les principales manifestations sont : érythrose du visage, couperose avec dilatations télangiectasiques et lésions papulo-pustuleuses. Les mécanismes provoquant l'apparition de la rosacée sont mal connus, les facteurs susceptibles de favoriser la rosacée sont nombreux. Pour plus d'information à ce sujet, on peut se reporter aux articles suivants : "Actualités sur la rosacée", H.N. Mouaci-Midoun, Abstract Dermato Hebdo n° 309, 27.01.1997, p. 15-19 ; "Rosacée : les traitements classiques", J.-M. Mazer, T. Fusade, Réalités thérapeutiques en Dermato-Vénérologie, N° 52, novembre 1995, p. 8-12.

Depuis plusieurs années, les dermatologues français utilisent en préparation magistrale le métronidazole dans la crème Physiane® commercialisée par le laboratoire La Roche Posay pour le traitement de la rosacée.

L'efficacité de ces préparations dans le traitement de la rosacée était attribuée au métronidazole (antiparasitaire) qui reste, avec les cyclines (antibiotique), l'une des molécules les plus prescrites dans le traitement de la rosacée quel que soit le support dans lequel il est incorporé.

Par ailleurs Physiane était jusqu'à présent recommandée comme crème hydratante en complément de traitements anti-acnéiques classiques. Toutefois, il n'y a pas de lien entre l'acné et la rosacée qui sont deux affections cutanées distinctes et la demanderesse s'était limitée à la prescrire pour son efficacité dans l'hydratation de la peau.

Les traitements actuels de la rosacée, pour être efficaces, sont souvent prescrits sur de longues périodes. Or, l'utilisation prolongée de molécules actives telles que des antibiotiques n'est pas souhaitable, en particulier parce qu'elle peut induire la résistance de certaines souches bactériennes à ces molécules. En outre, l'utilisation des cyclines et du métronidazole sont contre-indiqués chez la femme enceinte.

Par conséquent il subsiste le besoin d'un traitement de la rosacée qui puisse être utilisé par tous les sujets, de façon prolongée et sans effets secondaires.

Il a déjà été proposé dans la demande EP-0 775 492 de traiter différents désordres cutanés, dont la couperose qui est l'un des symptômes de la rosacée, par application topique d'une composition renfermant différents antagonistes de substance P. L'Exemple D illustre ainsi une composition renfermant un sel de baryum en association avec divers tensioactifs et corps gras.

Toutefois, il n'était pas prévisible à la lecture de ce document qu'une composition proche de celle exemplifiée mais ne comportant pas de sel de baryum serait aussi efficace contre la rosacée.

Or, c'est avec étonnement que la demanderesse a découvert que la crème Physiane® seule, sans métronidazole, et ne contenant pas de sel de baryum, était d'une efficacité équivalente aux traitements classiques à base de métronidazole ou de sels de baryum pour le traitement de la rosacée.

L'invention a donc pour objet l'utilisation pour la préparation d'une composition destinée à la prévention et/ou au traitement de la rosacée, d'une composition cosmétique comprenant au moins les constituants (i) à (vii) suivants, les pourcentages étant donnés en poids par rapport au poids total de la composition cosmétique :

(i) de 1 à 5 % d'au moins un acide gras en $C_{12}$-$C_{24}$ ;

(ii) de 5 à 15 % d'au moins un ester d'acide gras en $C_{12}$-$C_{24}$ et d'un fragment polyoxyde d'alkylène en $C_2$-$C_3$ comportant de 2 à 100 résidus oxyde d'alkylène

(iii) de 1 à 20 % d'au moins un glycéride d'acide gras en $C_{12}$-$C_{22}$ éventuellement polyoxyalkyléné comportant de 0 à 20 résidus oxyde d'alkyléne ;

(iv) de 1 à 20 % d'au moins un ester d'un acide gras en $C_{12}$-$C_{24}$ et d'un alcool en $C_1$-$C_6$ ;

(v) de 0,1 à 10 % de glycérol ;

(vi) de 0,1 à 3 % d'un alcool gras en $C_{12}$-$C_{24}$ ;

(vii) de 50 à 85 % d'eau ;

cette composition ne comprenant pas de métronidazole, ni de sel de baryum.

Une telle composition peut être appliquée de façon prolongée en toute innocuité, ce qui permet d'envisager son utilisation aussi bien pour le traitement de la rosacée, pour lequel on a illustré ci-dessous son efficacité, que pour la prévention de la rosacée, par une utilisation quotidienne en dehors des périodes d'apparition des symptômes.

L'invention a en outre pour objet l'utilisation d'une composition cosmétique telle que décrite ci-dessus pour la préparation d'une composition destinée à la prévention et/ou au traitement de la rosacée, dans le but de diminuer les papulo-pustules dues à la rosacée.

L'invention a également pour objet l'utilisation d'une composition cosmétique telle que décrite ci-dessus pour la préparation d'une composition destinée à la prévention et/ou au traitement de la rosacée, dans le but de réduire l'érythème associé à la rosacée.

L'invention a enfin pour objet l'utilisation d'une composition cosmétique telle que décrite ci-dessus pour la préparation d'une composition destinée à la prévention et/ou au traitement de la rosacée, dans le but de réduire les télangiectasies associées à la rosacée.

De préférence, la composition décrite ci-dessus vérifie l'une au moins, et de préférence toutes les conditions ci-dessous

- le constituant (i) est un acide gras en $C_{16}$-$C_{20}$, de préférence l'acide stéarique ;

- le constituant (i) est présent en quantité allant de 2 à 4 %, de préférence de 2,5 à 3,5 %, en poids par rapport au poids total de la composition ;

- le constituant (ii) est un ester d'acide gras en $C_{16}$-$C_{20}$, de préférence un ester d'acide stéarique ;

- le constituant (ii) est un ester d'acide gras et de polyoxyde d'éthylène ;

- le constituant (ii) est un mélange d'esters d'acides gras et de polyoxyde d'alkylène comportant, pour au moins un ester, de 2 à 15 résidus oxyde d'alkylène et, pour au moins un ester, de 20 à 50 résidus oxyde d'alkylène ;

- le constituant (ii) est un mélange d'esters d'acides gras et de polyoxyde d'alkylène, la balance HLB (définie ci-dessous) du mélange allant de 9 à 15, de préférence de 11 à 14 ;

- le constituant (ii) est présent en quantité allant de 7 à 13 %, de préférence de 9 à 11 %, en poids par rapport au poids total de la composition ;

- le constituant (iii) est un mélange d'au moins un glycéride d'acide gras en $C_{12}$-$C_{22}$ et d'au moins un glycéride d'acide gras en $C_{12}$-$C_{22}$ polyoxyéthyléné comportant de 2 à 20 résidus oxyde d'éthylène ;

- le constituant (iii) est un mélange d'huile de palme hydrogénée et d'huile de noyau de palme polyoxyéthyléné par 4 à 10 résidus oxyde d'éthylène ;

- le constituant (iii) est présent en quantités allant de 1 à 10 %, préférentiellement de 2 à 5 %, en poids par rapport au poids total de la composition ;

- le constituant (iv) est un ester d'acide gras en $C_{14}$-$C_{18}$, préférentiellement un ester d'acide palmitique ;

- le constituant (iv) est un ester d'acide gras et d'un alcool en $C_2$-$C_4$, de préférence un ester d'acide gras et d'alcool isopropylique

- le constituant (iv) est présent en quantité allant de 1 à 10 %, de préférence de 2 à 6 % en poids par rapport au poids total de la composition ;

- le constituant (v) est présent en quantité allant de 0,5 à 5 %, de préférence de 0,5 à 3 % en poids par rapport au poids total de la composition ;

- le constituant (vi) est un alcool gras en $C_{14}$-$C_{18}$, de préférence le constituant (vi) est l'alcool cétylique ;

- le constituant (vi) est présent en quantité allant de 0,1 à 1 %, de préférence de 0,2 à 0,8 % en poids par rapport au poids total de la composition ;

- le constituant (vii) est au moins en partie une eau minérale ou thermale, de préférence de l'eau de la Roche Posay ;

- le constituant (vii) est présent en quantité allant de 65 à 80 %, de préférence de 70 à 80 % en poids par rapport au poids total de la composition ;

- les constituants (iii) et (iv) représentent au total au moins 5 % en poids par rapport au poids total de la composition ;

- le pH de la composition est ajusté à une valeur allant de 4 à 7.

On calcule la balance HLB (balance hydrophile-lipophile) d'un émulsionnant suivant la formule suivante :

$$HLB = \frac{100-L}{5}$$

dans laquelle L représente le pourcentage en poids du groupement lipophile par rapport au poids de la molécule entière. La balance HLB d'un mélange d'émulsionnants est définie comme la moyenne des balances HLB de chacun des émulsionnants, pondérée par la quantité en poids de chacun des émulsionnants dans le mélange.

L'eau utilisée dans la composition peut être de tout type : eau déminéralisée, eau minérale, eau thermale ou leurs mélanges. En général, une eau minérale est propre à la consommation, ce qui n'est pas toujours le cas d'une eau thermale. Chacune de ces eaux contient, entre autre, des minéraux solubilisés et des oligo-éléments. Ces eaux sont connues pour être employées à des fins de traitement spécifique selon les oligo-éléments et les minéraux particuliers qu'elles contiennent, tels que l'hydratation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou oligo-élémentaires préparées à partir d'eau purifiée (déminéralisée ou distillée).

Une eau thermale ou minérale naturelle utilisée selon l'invention peut, par exemple être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene.

La composition comprend en outre de préférence au moins un dérivé de protéine animale ou végétale, de préférence un dérivé de protéine végétale, ce dérivé pouvant être choisi parmi les hydrolysats de protéines, les protéines greffées à leur extrémité azotée par un acide gras, les hydrolysats de protéines N-acylés par un résidu acide gras. Dans le cas d'un dérivé hydrolysé de protéine, l'hydrolyse peut être complète ou partielle.

La composition de l'invention peut se présenter sous toutes les formes galéniques normalement utilisées pour une application topique, notamment sous forme d'une émulsion huile-dans-eau ou eau-dans-huile ou d'une émulsion multiple, d'une dispersion d'huile dans une phase aqueuse à l'aide de sphérules, ces sphérules pouvant être des nanoparticules polymériques telles que les nanosphéres et les nanocapsules ou des vésicules lipidiques de type ionique et/ou non-ionique.

De préférence la composition de l'invention est sous la forme d'une émulsion huile-dans-eau.

De façon avantageuse, la composition de l'invention répond à l'ensemble des conditions suivantes :

- le constituant (i) est l'acide stéarique ;

- le constituant (i) est présent en quantité allant de 2,5 à 3,5 %, en poids par rapport au poids total de la composition ;

- le constituant (ii) est un mélange d'esters d'acide stéarique comportant, de 40 à 60% d'au moins un ester d'acide stéarique et d'oxyde d'éthylène comportant de 3 à 8 résidus oxyde d'éthylène et de 40 à 60% d'au moins un ester d'acide stéarique et d'oxyde d'éthylène comportant de 25 à 40 résidus oxyde d'alkylène ;

- le constituant (ii) est présent en quantité allant de 9 à 11 %, en poids par rapport au poids total de la composition ;

- le constituant (iii) est un mélange d'huile de palme hydrogénée et d'huile de noyau de palme polyoxyéthyléné par 4 à 10 résidus oxyde d'éthylène ;

- le constituant (iii) est présent en quantités allant de 2 à 5 %, en poids par rapport au poids total de la composition ;

- le constituant (iv) est un ester d'acide palmitique et d'alcool isopropylique ;

- le constituant (iv) est présent en quantité allant de 2 à % en poids par rapport au poids total de la composition ;

- le constituant (v) est présent en quantité allant de 0,8 à 1,5 % en poids par rapport au poids total de la composition

- le constituant (vi) est l'alcool cétylique ;

- le constituant (vi) est présent en quantité allant de 0,2 à 0,8 % en poids par rapport au poids total de la composition ;

- le constituant (vii) est au moins en partie de l'eau de la Roche Posay ;

- le constituant (vii) est présent en quantité allant de 70 à 80 % en poids par rapport au poids total de la composition ;

- les constituants (iii) et (iv) représentent au total au moins 5 % en poids par rapport au poids total de la composition ;

- le pH de la composition est ajusté à une valeur allant de 4 à 7 ;

- la composition est sous la forme d'une émulsion huile-dans-eau.

Cette composition peut être plus ou moins fluide et avoir l'aspect d'une crème blanche ou colorée, d'une pommade, d'un lait, d'une lotion, d'un sérum. Elle peut être utilisée comme produit de soin et/ou comme produit de maquillage.

De façon connue, la composition de l'invention peut contenir également les adjuvants habituels dans les domaines cosmétique et dermatologique, dès lors que ceux-ci ne réduisent pas l'efficacité de la composition vis-à-vis de la rosacée. On peut citer en particulier les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les vitamines, les agents kératolytiques et/ou desquamants (acide salicylique et ses dérivés, alpha-hydroxyacides, acide ascorbique et ses dérivés), les agents anti-inflammatoires, les agents apaisants et leurs mélanges. En cas d'incompatibilité, ces actifs peuvent être incorporés dans des sphérules, notamment des vésicules ioniques ou non-ioniques et/ou des nanoparticules (nanocapsules et/ou nanosphères), de manière à les isoler les uns des autres dans la composition.

Les exemples suivants sont donnés à titre d'illustration de l'invention. Dans les exemples, les pourcentages sont donnés en poids par rapport au poids total de la composition.

**Exemple 1 :**

| | |
|---|---|
| Acide stéarique | 3 % |
| Stéarate de PEG.32 | 5 % |
| Stéarate de PEG.6 | 5 % |
| Huile de palme hydrogénée /Ester d'huile de noyau de palme et de PEG-6 | 3 % |
| Palmitate d'isopropyle | 4 % |
| Glycerine | 1 % |
| Alcool cétylique | 0,5 % |
| Acide citrique anhydre | 0,1 % |
| Conservateurs | qs |

(suite)

| Eau | qsp 100 |
| --- | --- |

On prépare une émulsion huile-dans-eau à l'aide des constituants énumérés ci-dessus.

**Exemple 2 (comparatif) :**

| Acide Stéarique | 3 % |
| --- | --- |
| Stéarate de PEG.32 | 5 % |
| Stéarate de PEG.6 | 5 % |
| Huile de palme hydrogénée /Ester d'huile de noyau de palme et de PEG-6 | 3 % |
| Palmitate d'isopropyle | 4 % |
| Glycerine | 1 % |
| Alcool cétylique | 0,5 % |
| Acide citrique anhydre | 0,1 % |
| Metronidazole | 0,75 % |
| Conservateurs | qs |
| Eau | qsp 100 |

Dans un essai contrôle, réalisé en double aveugle sur 2 groupes parallèles, 95 patients atteints de rosacée ont appliqué l'une ou l'autre de ces crèmes, deux fois par jour (matin et soir) après la toilette, selon une randomisation (distribution aléatoire) préétablie avant le début de l'étude. L'investigateur a déterminé à J0, J28 et J56 le nombre des papulopustules et, parallèlement, l'efficacité du produit. Les résultats suivants ont été obtenus :

Variation relative du nombre de papulopustules entre J0, J28 et J56

| | $\dfrac{J0-J28}{J0}$ | $\dfrac{J0-J56}{J0}$ |
| --- | --- | --- |
| **Composition 1** | 26 % | 41 % |
| **Composition 2** | 36 % | 46% |

Il n'y a pas de différence significative entre les deux traitements.

Efficacité selon l'expérimentateur

- à J28

| | PATIENTS % | |
|---|---|---|
| | Composition 1 | Composition 2 |
| Etat aggravé | 14 % | 7 % |
| Etat stable | 30 % | 34 % |
| Rosacée améliorée ou guérie | 56 % | 59 % |
| Diminution de l'érythème | 33 % | 50 % |
| Diminution des télangiectasies | 6 % | 25 % |

- à J56

| | PATIENTS % | |
|---|---|---|
| | Composition 1 | Composition 2 |
| Etat aggravé | 8 % | 8 % |
| Etat stable | 42 % | 37 % |
| Rosacée améliorée ou guérie | 50 % | 55 % |

| | Composition 1 | Composition 2 |
|---|---|---|
| Diminution de l'érythème | 29 % | 45 % |
| Diminution des télangiectasies | 17 % | 29 % |

Les différences entre la crème selon l'invention et la crème au métronidazole ne sont pas significatives sur les 3 premiers critères.

Sur l'érythème et les télangiectasies, la crème selon l'invention présente une efficacité inférieure à celle de la crème au métronidazole, mais une efficacité toutefois significative.

En outre, on a observé la même tolérance (72 % de bonne ou très bonne tolérance) pour les deux crèmes.

**Revendications**

1. Utilisation pour la préparation d'une composition destinée à la prévention et/ou au traitement de la rosacée, d'une composition cosmétique comprenant au moins les constituants (i) à (vii) suivants, les pourcentages étant donnés en poids par rapport au poids total de la composition :

(i) de 1 à 5 % d'au moins un acide gras en $C_{12}$-$C_{24}$ ;

(ii) de 5 à 15 % d'au moins un ester d'acide gras en $C_{12}$-$C_{24}$ et d'un fragment polyoxyde d'alkylène en $C_2$-$C_3$ comportant de 2 à 100 résidus oxyde d'alkylène ;

(iii) de 1 à 20 % d'au moins un glycéride d'acide gras en $C_{12}$-$C_{22}$ éventuellement polyoxyalkyléné comportant de 0 à 20 résidus oxyde d'alkylène ;

(iv) de 1 à 20 % d'au moins un ester d'un acide gras en $C_{12}$-$C_{24}$ et d'un alcool en $C_1$-$C_6$ ;

(v) de 0,1 à 10 % de glycérol ;

(vi) de 0,1 à 3 % d'un alcool gras en $C_{12}$-$C_{24}$ ;

(vii) de 50 à 85 % d'eau ;

cette composition ne comprenant pas de métronidazole, ni de sel de baryum.

2. Utilisation selon la revendication 1, dans le but de diminuer les papulo-pustules dues à la rosacée.

3. Utilisation selon la revendication 1, dans le but de réduire l'érythème associé à la rosacée.

4. Utilisation selon la revendication 1, dans le but de réduire les télangiectasies associées à la rosacée.

5. Utilisation selon l'une quelconque des revendications 1 à 4, **caractérisée en ce que** la composition cosmétique décrite ci-dessus vérifie l'une au moins, et de préférence toutes les conditions ci-dessous :

   - le constituant (i) est un acide gras en $C_{16}$-$C_{20}$, de préférence l'acide stéarique ;

   - le constituant (i) est présent en quantité allant de 2 à 4 %, de préférence de 2,5 à 3,5 %, en poids par rapport au poids total de la composition ;

   - le constituant (ii) est un ester d'acide gras en $C_{16}$-$C_{20}$, de préférence un ester d'acide stéarique ;

   - le constituant (ii) est un ester d'acide gras et de polyoxyde d'éthylène ;

   - le constituant (ii) est un mélange d'esters d'acides gras et de polyoxyde d'alkylène comportant, pour au moins un ester, de 2 à 15 résidus oxyde d'alkylène et, pour au moins un ester, de 20 à 50 résidus oxyde d'alkylène ;

   - le constituant (ii) est un mélange d'esters d'acides gras et de polyoxyde d'alkylène, la balance HLB du mélange allant de 9 à 15, de préférence de 11 à 14 ;

   - le constituant (ii) est présent en quantité allant de 7 à 13 %, de préférence de 9 à 11 %, en poids par rapport au poids total de la composition ;

   - le constituant (iii) est un mélange d'au moins un glycéride d'acide gras en $C_{12}$-$C_{22}$ et d'au moins un glycéride d'acide gras en $C_{12}$-$C_{22}$ polyoxyéthyléné comportant de 2 à 20 résidus oxyde d'éthylène ;

   - le constituant (iii) est un mélange d'huile de palme hydrogénée et d'huile de noyau de palme polyoxyéthyléné par 4 à 10 résidus oxyde d'éthylène ;

   - le constituant (iii) est présent en quantités allant de 1 à 10 %, préférentiellement de 2 à 5 %, en poids par rapport au poids total de la composition ;

   - le constituant (iv) est un ester d'acide gras en $C_{14}$-$C_{18}$, préférentiellement un ester d'acide palmitique ;

   - le constituant (iv) est un ester d'acide gras et d'un alcool en $C_2$-$C_4$, de préférence un ester d'acide gras et d'alcool isopropylique ;

   - le constituant (iv) est présent en quantité allant de 1 à 10 %, de préférence de 2 à 6 % en poids par rapport au poids total de la composition ;

   - le constituant (v) est présent en quantité allant de 0,5 à 5 %, de préférence de 0,5 à 3 % en poids par rapport au poids total de la composition ;

   - le constituant (vi) est un alcool gras en $C_{14}$-$C_{18}$, de préférence le constituant (vi) est l'alcool cétylique ;

   - le constituant (vi) est présent en quantité allant de 0,1 à 1 %, de préférence de 0,2 à 0,8 % en poids par rapport au poids total de la composition ;

   - le constituant (vii) est au moins en partie une eau minérale ou thermale, de préférence de l'eau de la Roche Posay ;

   - le constituant (vii) est présent en quantité allant de 65 à 80 %, de préférence de 70 à 80 % en poids par rapport au poids total de la composition ;

- les constituants (iii) et (iv) représentent au total au moins 5 % en poids par rapport au poids total de la composition ;

- le pH de la composition est ajusté à une valeur allant de 4 à 7.

6. Utilisation selon l'une quelconque des revendications 1 à 5, **caractérisée en ce que** la composition comprend en outre au moins un dérivé de protéine animale ou végétale.

7. Utilisation selon l'une quelconque des revendications 1 à 6, **caractérisée en ce que** la composition cosmétique de l'invention est sous la forme d'une émulsion huile-dans-eau.

8. Utilisation selon l'une quelconque des revendications 1 à 7, **caractérisée par le fait que** la composition cosmétique répond à l'ensemble des conditions suivantes :

- le constituant (i) est l'acide stéarique ;

- le constituant (i) est présent en quantité allant de 2,5 à 3,5 %, en poids par rapport au poids total de la composition ;

- le constituant (ii) est un mélange d'esters d'acide stéarique comportant, de 40 à 60% d'au moins un ester d'acide stéarique et d'oxyde d'éthylène comportant de 3 à 8 résidus oxyde d'éthylène et de 40 à 60% d'au moins un ester d'acide stéarique et d'oxyde d'éthylène comportant de 25 à 40 résidus oxyde d'alkylène ;

- le constituant (ii) est présent en quantité allant de 9 à 11 %, en poids par rapport au poids total de la composition ;

- le constituant (iii) est un mélange d'huile de palme hydrogénée et d'huile de noyau de palme polyoxyéthyléné par 4 à 10 résidus oxyde d'éthylène ;

- le constituant (iii) est présent en quantités allant de 2 à 5 %, en poids par rapport au poids total de la composition ;

- le constituant (iv) est un ester d'acide palmitique et d'alcool isopropylique ;

- le constituant (iv) est présent en quantité allant de 2 à 6 % en poids par rapport au poids total de la composition ;

- le constituant (v) est présent en quantité allant de 0,8 à 1,5 % en poids par rapport au poids total de la composition ;

- le constituant (vi) est l'alcool cétylique ;

- le constituant (vi) est présent en quantité allant de 0,2 à 0,8 % en poids par rapport au poids total de la composition ;

- le constituant (vii) est au moins en partie de l'eau de la Roche Posay ;

- le constituant (vii) est présent en quantité allant de 70 à 80 % en poids par rapport au poids total de la composition ;

- les constituants (iii) et (iv) représentent au total au moins 5 % en poids par rapport au poids total de la composition ;

- le pH de la composition est ajusté à une valeur allant de 4 à 7 ;

- la composition est sous la forme d'une émulsion huile-dans-eau.

**Patentansprüche**

1. Verwendung einer kosmetischen Zusammensetzung für die Herstellung einer Zusammensetzung, die für die Vorbeugung vor und/oder die Behandlung der Rosacea vorgesehen ist, wobei die kosmetische Zusammensetzung mindestens die folgenden Bestandteile (i) bis (vii) enthält, wobei die Prozentangaben Angaben in Gewichtsprozent sind, bezogen auf das Gesamtgewicht der Zusammensetzung:

   (i) 1 bis 5 % mindestens einer $C_{12-24}$-Fettsäure;
   (ii) 5 bis 15 % mindestens eines Esters aus einer $C_{12-24}$-Fettsäure und einem Poly-$(C_2\text{-}C_3)$-alkylenoxid-Fragment, das 2 bis 100 Alkylenoxid-Reste enthält;
   (iii) 1 bis 20 % mindestens eines $C_{12-22}$-Fettsäureglycerids, der gegebenenfalls polyalkoxyliert ist, das 0 bis 20 Alkylenoxid-Reste enthält;
   (iv) 1 bis 20 % mindestens eines Esters aus einer $C_{12-24}$-Fettsäure und einem $C_{1-6}$-Alkohol;
   (v) 0,1 bis 10 % Glycerin;
   (vi) 0,1 bis 3 % eines $C_{12-24}$-Fettalkohols;
   (vii) 50 bis 85 % Wasser,

   wobei diese Zusammensetzung weder Metronidazol noch ein Bariumsalz enthält.

2. Verwendung nach Anspruch 1 mit dem Ziel, die durch die Rosacea hervorgerufenen Papulopusteln zu vermindern.

3. Verwendung nach Anspruch 1 mit dem Ziel, das mit der Rosacea zusammenhängende Erythem zu verringern.

4. Verwendung nach Anspruch 1 mit dem Ziel, die mit der Rosacea zusammenhängenden Teleangiektasien zu verringern.

5. Verwendung nach einem der Ansprüche 1 bis 4, **dadurch gekennzeichnet, dass** die oben beschriebene kosmetische Zusammensetzung mindestens eine der und vorzugsweise alle folgenden Bedingungen erfüllt:

   - der Bestandteil (i) ist eine $C_{16-20}$-Fettsäure, vorzugsweise Stearinsäure,
   - der Bestandteil (i) ist in einer Menge enthalten, die im Bereich von 2 bis 4 Gew.-%, vorzugsweise 2,5 bis 3,5 Gew.-%, bezogen auf das Gesamtgewicht der Zusammensetzung, liegt;
   - der Bestandteil (ii) ist ein Ester einer $C_{16-20}$-Fettsäure, vorzugsweise ein Stearinsäureester;
   - der Bestandteil (ii) ist ein Ester aus einer Fettsäure und Polyethylenoxid;
   - der Bestandteil (ii) ist ein Gemisch von Estern aus Fettsäuren und Polyalkylenoxid, wobei mindestens ein Ester 2 bis 15 Alkylenoxid-Reste und mindestens ein Ester 20 bis 50 Alkylenoxid-Reste enthält;
   - der Bestandteil (ii) ist ein Gemisch von Estern aus Fettsäuren und Polyalkylenoxid, wobei der HLB-Wert (hydrophilic lipophilic balance) des Gemischs im Bereich von 9 bis 15 und vorzugsweise 11 bis 14 liegt;
   - der Bestandteil (ii) ist in einer Menge enthalten, die im Bereich von 7 bis 13 Gew.-% und vorzugsweise 9 bis 11 Gew.-%, bezogen auf das Gesamtgewicht der Zusammensetzung, liegt;
   - der Bestandteil (iii) ist ein Gemisch aus mindestens einem $C_{12}\text{-}C_{22}$-Fettsäureglycerid und mindestens einem polyethoxylierten $C_{12}\text{-}C_{22}$-Fettsäureglycerid, das 2 bis 20 Ethylenoxid-Reste enthält;
   - der Bestandteil (iii) ist ein Gemisch aus hydriertem Palmöl und Palmkemöl, das mit 4 bis 10 Ethylenoxid-Resten polyethoxyliert ist;
   - der Bestandteil (iii) ist in einer Menge enthalten, die im Bereich von 1 bis 10 Gew.-%, vorzugsweise 2 bis 5 Gew.-%, bezogen auf das Gesamtgewicht der Zusammensetzung, liegt;
   - der Bestandteil (iv) ist ein Ester einer $C_{14-18}$-Fettsäure, vorzugsweise ein Palmitinsäureester;
   - der Bestandteil (iv) ist ein Ester aus einer Fettsäure und einem $C_{2-4}$-Alkohol, vorzugsweise ein Ester aus einer Fettsäure und Isopropylalkohol;
   - der Bestandteil (iv) ist in einer Menge enthalten, die im Bereich von 1 bis 10 Gew.-%, vorzugsweise 2 bis 6 Gew.-%, bezogen auf das Gesamtgewicht der Zusammensetzung, liegt;
   - der Bestandteil (v) ist in einer Menge enthalten, die im Bereich von 0,5 bis 5 Gew.-%, vorzugsweise 0,5 bis 3 Gew.-%, bezogen auf das Gesamtgewicht der Zusammensetzung, liegt;
   - der Bestandteil (vi) ist ein $C_{14-18}$-Fettalkohol, und vorzugsweise handelt es sich bei dem Bestandteil (vi) um Cetylalkohol;
   - der Bestandteil (vi) ist in einer Menge enthalten, die im Bereich von 0,1 bis 1 Gew.-%, vorzugsweise 0,2 bis 0,8 Gew.-%, bezogen auf das Gesamtgewicht der Zusammensetzung, liegt;
   - der Bestandteil (vii) besteht zumindest teilweise aus einem Mineral- oder Thermalwasser, vorzugsweise La-Roche-Posay-Wasser;

- der Bestandteil (vii) ist in einer Menge enthalten, die im Bereich von 65 bis 80 Gew.-%, vorzugsweise 70 bis 80 Gew.-%, bezogen auf das Gesamtgewicht der Zusammensetzung, liegt;
- die Bestandteile (iii) und (iv) machen insgesamt mindestens 5 Gew.-%, bezogen auf das Gesamtgewicht der Zusammensetzung, aus;
- der pH-Wert der Zusammensetzung ist auf einen Wert eingestellt, der im Bereich von 4 bis 7 liegt.

6. Verwendung nach einem der Ansprüche 1 bis 5, **dadurch gekennzeichnet, dass** die Zusammensetzung außerdem mindestens ein Derivat eines tierischen oder pflanzlichen Proteins enthält.

7. Verwendung nach einem der Ansprüche 1 bis 6, **dadurch gekennzeichnet, dass** die erfindungagemäße kosmetische Zusammensetzung in Form einer Öl-in-Wasser-Emulsion vorliegt.

8. Verwendung nach einem der Ansprüche 1 bis 7, **dadurch gekennzeichnet, dass** die kosmetische Zusammensetzung alle folgenden Bedingungen erfüllt:

- bei dem Bestandteil (i) handelt es sich um Stearinsäure;
- der Bestandteil (i) ist in einer Menge enthalten, die im Bereich von 2,5 bis 3,5 Gew.-%, bezogen auf das Gesamtgewicht der Zusammensetzung, liegt;
- der Bestandteil (ii) ist ein Gemisch von Stearinsäureestern, das enthält: 40 bis 60 % mindestens eines Esters aus Stearinsäure und Ethylenoxid, der 3 bis 8 Ethylenoxid-Reste enthält, und 40 bis 60 % mindestens eines Esters aus Stearinsäure und Ethylenoxid, der 25 bis 40 Alkylenoxid-Reste enthält;
- der Bestandteil (ii) ist in einer Menge enthalten, die im Bereich von 9 bis 11 Gew.-%, bezogen auf das Gesamtgewicht der Zusammensetzung, liegt;
- der Bestandteil (iii) ist ein Gemisch aus hydriertem Palmöl und Palmkemöl, das mit 4 bis 10 Ethylenoxid-Resten polyethoxyliert ist;
- der Bestandteil (iii) ist in Mengen enthalten, die im Bereich von 2 bis 5 Gew.%, bezogen auf das Gesamtgewicht der Zusammensetzung, liegen;
- der Bestandteil (iv) ist ein Ester aus Palmitinsäure und Isopropylalkohol;
- der Bestandteil (iv) ist in einer Menge enthalten, die im Bereich von 2 bis 6 Gew-%, bezogen auf das Gesamtgewicht der Zusammensetzung, liegt;
- der Bestandteil (v) ist in einer Menge enthalten, die im Bereich von 0,8 bis 1,5 Gew.-%, bezogen auf das Gesamtgewicht der Zusammensetzung, liegt;
- bei dem Bestandteil (vi) handelt es sich um Cetylalkohol;
- der Bestandteil (vi) ist in einer Menge enthalten, die im Bereich von 0,2 bis 0,8 Gew.-%, bezogen auf das Gesamtgewicht der Zusammensetzung, liegt;
- der Bestandteil (vii) besteht zumindest teilweise aus La-Roche-Posay-Wasser;
- der Bestandteil (vii) ist in einer Menge enthalten, die im Bereich von 70 bis 80 Gew.-%, bezogen auf das Gesamtgewicht der Zusammensetzung, liegt:

- die Bestandteile (iii) und (iv) machen insgesamt mindestens 5 Gew.-% aus, bezogen auf das Gesamtgewicht der Zusammensetzung;
- der pH-Wert der Zusammensetzung ist auf einen Wert eingestellt, der im Bereich von 4 bis 7 liegt;
- die Zusammensetzung liegt in Form einer Öl-in-Wasser-Emulsion vor.

**Claims**

1. Use for the preparation of a composition intended to prevent and/or treat rosacea, of a cosmetic composition comprising at least the following constituents (i) to (vii), the percentages being given by weight relative to the total weight of the composition:

(i) from 1 to 5% of at least one $C_{12}$-$C_{24}$ fatty acid;
(ii) from 5 to 15% of at least one ester of $C_{12}$-$C_{24}$ fatty acid and of a $C_2$-$C_3$ polyalkylene oxide fragment containing from 2 to 100 alkylene oxide residues;
(iii) from 1 to 20% of at least one optionally polyoxyalkylenated $C_{12}$-$C_{22}$ fatty acid glyceride containing from 0 to 20 alkylene oxide residues;
(iv) from 1 to 20% of at least one ester of a $C_{12}$-$C_{24}$ fatty acid and of a $C_1$-$C_6$ alcohol;

(v) from 0.1 to 10% of glycerol;
(vi) from 0.1 to 3% of a $C_{12}$-$C_{24}$ fatty alcohol;
(vii) from 50 to 85% of water;

this composition not comprising any metronidazole or any barium salt.

**2.** Use according'to Claim 1, with the aim of reducing the papulo-pustules due to rosacea.

**3.** Use according to Claim 1, with the aim of reducing the erythema associated with rosacea.

**4.** Use according to Claim 1, with the aim of reducing the telangiectasia associated with rosacea.

**5.** Use according to any one of Claims 1 to 4, **characterized in that** the cosmetic composition described above satisfies at least one, and preferably all, of the conditions below:

- the constituent (i) is a $C_{16}$-$C_{20}$ fatty acid, preferably stearic acid;
- the constituent (i) is present in an amount ranging from 2 to 4%, preferably from 2.5 to 3.5%, by weight relative to the total weight of the composition;
- the constituent (ii) is a $C_{16}$-$C_{20}$ fatty acid ester, preferably a stearic acid ester;
- the constituent (ii) is a fatty acid ester of polyethylene oxide;
- the constituent (ii) is a mixture of esters of fatty acids and of polyalkylene oxide containing, for at least one ester, from 2 to 15 alkylene oxide residues, and, for at least one ester, from 20 to 50 alkylene oxide residues;
- the constituent (ii) is a mixture of fatty acid esters of polyalkylene oxide, the HLB value of the mixture ranging from 9 to 15, preferably from 11 to 14;
- the constituent (ii) is present in an amount ranging from 7 to 13%, preferably from 9 to 11%, by weight relative to the total weight of the composition;
- the constituent (iii) is a mixture of at least one $C_{12}$-$C_{22}$ fatty acid glyceride and of at least one polyoxyethylenated $C_{12}$-$C_{22}$ fatty acid glyceride containing from 2 to 20 ethylene oxide residues;
- the constituent (iii) is a mixture of hydrogenated palm oil and of palm kernel oil polyoxyethylenated with 4 to 10 ethylene oxide residues;
- the constituent (iii) is present in amounts ranging from 1 to 10%, preferably from 2 to 5%, by weight relative to the total weight of the composition;
- the constituent (iv) is a $C_{14}$-$C_{18}$ fatty acid ester, preferably a palmitic acid ester;
- the constituent (iv) is a fatty acid ester of a $C_2$-$C_4$ alcohol, preferably a fatty acid ester of isopropyl alcohol;
- the constituent (iv) is present in an amount ranging from 1 to 10%, preferably from 2 to 6%, by weight relative to the total weight of the composition;
- the constituent (v) is present in an amount ranging from 0.5 to 5%, preferably from 0.5 to 3%, by weight relative to the total weight of the composition;
- the constituent (vi) is a $C_{14}$-$C_{18}$ fatty alcohol, preferably cetyl alcohol;
- the constituent (vi) is present in an amount ranging from 0.1 to 1%, preferably from 0.2 to 0.8%, by weight relative to the total weight of the composition;
- the constituent (vii) is at least partly a mineral or spring water, preferably eau de la Roche Posay;
- the constituent (vii) is present in an amount ranging from 65 to 80%, preferably from 70 to 80%, by weight relative to the total weight of the composition;
- the constituents (iii) and (iv) represent in total at least 5% by weight relative to the total weight of the composition;
- the pH of the composition is adjusted to a value ranging from 4 to 7.

**6.** Use according to any one of Claims 1 to 5, **characterized in that** the composition also comprises at least one animal or plant protein derivative.

**7.** Use according to any one of Claims 1 to 6, **characterized in that** the cosmetic composition of the invention is in the form of an oil-in-water emulsion

**8.** Use according to any one of Claims 1 to 7, **characterized in that** the cosmetic composition satisfies all of the following conditions:

- the constituent (i) is stearic acid;

- the constituent (i) is present in an amount ranging from 2.5 to 3.5% by weight relative to the total weight of the composition;
- the constituent (ii) is a mixture of stearic acid esters containing from 40 to 60% of at least one stearic acid ester of ethylene oxide containing from 3 to 8 ethylene oxide residues and from 40 to 60% of at least one stearic acid ester of ethylene oxide containing from 25 to 40 alkylene oxide residues;
- the constituent (ii) is present in an amount ranging from 9 to 11% by weight relative to the total weight of the composition;
- the constituent (iii) is a mixture of hydrogenated palm oil and of palm kernel oil polyoxyethylenated with 4 to 10 ethylene oxide residues;
- the constituent (iii) is present in amounts ranging from 2 to 5% by weight relative to the total weight of the composition;
- the constituent (iv) is a palmitic acid ester of isopropyl alcohol; the constituent (iv) is present in an amount of from 2 to 6% by weight relative to the total weight of the composition;
- the constituent (v) is present in an amount ranging from 0.8 to 1.5% by weight relative to the total weight of the composition;
- the constituent (vi) is cetyl alcohol;
- the constituent (vi) is present in an amount ranging from 0.2 to 0.8% by weight relative to the total weight of the composition;
- the constituent (vii) is at least partly eau de la Roche Posay ;
- the constituent (vii) is present in an amount ranging from 70 to 30% by weight relative to the total weight of the composition ;
- the constituents (iii) and (iv) represent in total at least 5% by weight relative to the total weight of the composition ;
- the pH of the composition is adjusted to a value ranging from 4 to 7;
- the composition is in the form of an oil-in-water emulsion.